# EUROPEAN PATENT SPECIFICATION

(11) **EP 2 935 306 B1**
(45) Date of publication and mention of the grant of the patent: **30.11.2016**
(21) Application number: 13808024.7
(22) Date of filing: 18.12.2013
(51) Int. Cl.: C07H 21/00, C12Q 1/68

(54) **LABELED OLIGONUCLEOTIDE PROBES USED FOR NUCLEIC ACID SEQUENCE ANALYSIS**
MARKIERTE OLIGONUKLEOTIDSONDEN ZUR SEQUENZANALYSE VON NUKLEINSÄUREN
SONDES OLIGONUCLÉOTIDIQUES MARQUÉES UTILISÉES POUR ANALYSER DES SÉQUENCES D'ACIDES NUCLÉIQUES

(30) Priority: 20.12.2012 US 201261740297 P
(43) Date of publication of application: 28.10.2015
(73) Proprietor: Roche Diagnostics GmbH, 68305 Mannheim (DE); F.Hoffmann-La Roche AG, 4070 Basel (CH)
(72) Inventor: ANACLETO, Concordio, Dublin, California 94568 (US); BUGAWAN, Teodorica, Castro Valley, CA 94552 (US); SCHOENBRUNNER, Nancy, Charlestown, MA 02129 (US)
(74) Representative: Poredda, Andreas
(86) International application number: PCT/EP2013/077016
(87) International publication number: WO 2014/095952

(56) References cited:
- WO-A1-2009/038548
- WO-A2-03/102239
- US-B1- 6 184 379
- JU J ET AL: "FLUORESCENCE ENERGY TRANSFER DYE-LABELED PRIMERS FOR DNA SEQUENCINGAND ANALYSIS", PROCEEDINGS OF THE NATIONAL ACADEMY OF SCIENCES, NATIONAL ACADEMY OF SCIENCES, US, vol. 92, 1 May 1995 (1995-05-01), pages 4347-4351, XP002052420, ISSN: 0027-8424, DOI: 10.1073/PNAS.92.10.4347
- CATERINA CHIANELLA ET AL: "andgenetic variation in Alzheimerâ s disease patients treated with cholinesterase inhibitors", EUROPEAN JOURNAL OF CLINICAL PHARMACOLOGY, SPRINGER, BERLIN, DE, vol. 67, no. 11, 1 June 2011 (2011-06-01), pages 1147-1157, XP019964147, ISSN: 1432-1041, DOI: 10.1007/S00228-011-1064-X

## Description

### FIELD OF THE INVENTION

The invention relates to methods for the detection and analysis of nucleic acid sequences by use of fluorescently-labeled oligonucleotides. The invention has applications for genotyping, pathogen detection and *in vitro* diagnostics.

### BACKGROUND OF THE INVENTION

The development of nucleic acid amplification technology has revolutionized genetic analysis and engineering science. For example, the polymerase chain reaction (PCR) is commonly utilized to amplify specific target nucleic acids using selected primer nucleic acids, e.g., to facilitate the detection of target nucleic acid as part of a diagnostic, forensic or other application. Primers typically function in pairs that are designed for extension towards each other to cover the selected target region. A typical PCR cycle includes a high temperature (e.g., 85°C or more) denaturation step during which the strands of double-stranded nucleic acids separate from one another, a low temperature (e.g., 45-65°C) annealing step during which the primers hybridize to the separated single strand, and an intermediate temperature (e.g., around 72°C) extension step during which a nucleic acid polymerase extends the primers. Two-temperature thermocycling procedures are also utilized. These generally include a high temperature denaturation step and a low temperature anneal-extend step.

Various strategies for detecting amplification products have been developed and one of the most widely used method is the 5' nuclease or TaqMan^{®} assay. The 5' nuclease assay typically utilizes the 5' to 3' nuclease activity of certain DNA polymerases to cleave 5' nuclease oligonucleotide probes during the course of PCR. This assay allows for both the amplification of a target and the release of labels for detection, generally without resort to multiple handling steps of amplified products. The 5' nuclease probes typically include labeling moieties, such as a fluorescent reporter dye and a quencher dye. When the probe is intact, the proximity of the reporter dye to the quencher dye generally results in the suppression of the reporter fluorescence. During a 5' nuclease reaction, cleavage of the probe separates the reporter dye and the quencher dye from one another, resulting in a detectable increase in fluorescence from the reporter. The accumulation of PCR products or amplicons is typically detected indirectly by monitoring this increase in fluorescence in real time.

The TaqMan^{®} technology can also be used for DNA amplification and genotype detection in a single-step assay. In this format, two oligonucleotide probes are used, one for each allele, which are designed to hybridize to a region of the DNA template that contains the allele-specific nucleotide. The probes are each labeled with a different fluorescent dye. During the amplification step of PCR, the probe that is perfectly matched to the template is digested, resulting in an increase in fluorescence signal from the corresponding dye. However, the probe that has a single nucleotide mismatch cannot be stably annealed to the template DNA, and not be degraded by the nuclease activity. The corresponding reporter dye from the "mismatch" probe would still be quenched by the quencher molecule and exhibit no fluorescent signal. With the availability of more sophisticated fluorescence detection instruments, genotyping of multiple genes or of multiple allelic positions of one gene can be performed in this kind of assay by using employing multiple probes, each labeled with each unique fluorescent reporter dye.

WO 2009/038548 discloses oligonucleotides containing a labeled fluorescent reporter dye and a fluorescence quencher molecule and, e.g., describes a process in which a nucleic acid is labeled with a rhodamine dye in the 3' position. The process disclosed comprises the steps of providing a rhodamine dye, attaching a linker to it and coupling the dye via the linker to the oligonucleotide. The labeled nucleotide is used as a probe for detecting a target nucleic acid in amplification reactions. Appropriate labeled oligonucleotides have, however, a poor storage stability resulting in a huge decline in the fluorescence signals in growth curves generated by the probe.

### SUMMARY OF THE INVENTION

The present invention is directed to methods for generating a labeled oligonucleotide probe that contains a fluorescent rhodamine-derived dye for use in PCR reactions to detect a target nucleic acid. In a first aspect, the invention relates to a method of making a labeled oligonucleotide for use as a hybridization probe in a PCR reaction comprising:
providing a fluorescent dye that is a rhodamine derivative wherein said fluorescent dye is attached with a bifunctional linker molecule; binding a reactive group to the fluorescent dye via the bifunctional linker molecule for incorporating said fluorescent dye within the oligonucleotide;
incorporating the fluorescent dye between two internal nucleotides of the oligonucleotide, with the proviso that the fluorescent dye is not incorporated at the 5' terminus of the oligonucleotide, wherein the bifunctional linker molecule is consisting of L-threoninol or (2R,3R)-2-amino-1,3-butanediol.

In a second aspect, the invention relates to a method for detecting the presence or absence of a target nucleic acid or a target allele of a nucleic acid in a test sample, comprising:
performing a PCR reaction with the use of a labeled probe oligonucleotide that hybridizes with the target nucleic acid, wherein the labeled probe oligonucleotide is characterized by having: a fluorescent dye that is a rhodamine derivative which is attached with a bifunctional linker molecule; and a reactive group bound to the bifunctional linker molecule for incorporating the fluorescent dye between two internal nucleotides of the oligonucleotide, with the proviso that the fluorescent dye is not incorporated at the 5' terminus of the oligonucleotide, wherein the bifunction linker molecule is consisting of L-threoninol or (2R,3R)-2-amino-1,3-butanediol; detecting the signal from the fluorescent dye wherein the intensity of said signal represents the presence or absence of the target nucleic acid.

### BRIEF DESCRIPTION OF THE DRAWINGS

**Figure 1** shows the chemical structures of (A) rhodamine dye core and (B) L-threoninol.
**Figure 2** shows the chemical structure of JA270 (carboxylic acid analog)
**Figure 3** shows the elution pattern of the oligonucleotides in a UPLC column in the 5-dye reference reagent (5 dye mixture, 2 µM mixture) that was run in the morning (top) and in the evening (bottom) of the same day.
**Figure 4** shows the UPLC analysis of the 5-dye reference reagent that contains internal labeled JA270 oligonucleotide using fluorescence detection.
**Figure 5** shows the oligonucleotides present in the "mastermix" solution, as analyzed by UPLC.
**Figure 6** shows the oligonucleotides present in the same "mastermix" solution after 3 days storage at 4°C, as analyzed by UPLC.
**Figure 7** shows the PCR growth curves generated using the R33 probe that had been stored for 0 days, 3 weeks or 6 weeks with the positive control plasmid template (RR) or the negative control plasmid template (CC).

### DETAILED DESCRIPTION OF THE INVENTION

### Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and

Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

The term "nucleic acid" refers to polymers of nucleotides (e.g., ribonucleotides, deoxyribonucleotides, nucleotide analogs etc.) and comprising deoxyribonucleic acids (DNA), ribonucleic acids (RNA), DNA-RNA hybrids, oligonucleotides, polynucleotides, aptamers, peptide nucleic acids (PNAs), PNA-DNA conjugates, PNA-RNA conjugates, etc., that comprise nucleotides covalently linked together, either in a linear or branched fashion. A nucleic acid is typically single-stranded or double-stranded and will generally contain phosphodiester bonds, although in some cases, nucleic acid analogs are included that may have alternate backbones, including, for example, phosphoramide (Beaucage et al. (1993) Tetrahedron 49(10):1925); phosphorothioate (Mag et al. (1991) Nucleic Acids Res. 19:1437; and U.S. Pat. No. 5,644,048), phosphorodithioate (Briu et al. (1989) J. Am. Chem. Soc. 111:2321), O-methylphophoroamidite linkages (see Eckstein, Oligonucleotides and Analogues: A Practical Approach, Oxford University Press (1992)), and peptide nucleic acid backbones and linkages (see, Egholm (1992) J. Am. Chem. Soc. 114:1895). Other analog nucleic acids include those with positively charged backbones (Denpcy et al. (1995) Proc. Natl. Acad. Sci. USA 92: 6097); non-ionic backbones (U.S. Pat. Nos. 5,386,023, 5,637,684, 5,602,240, 5,216,141 and 4,469,863) and non-ribose backbones, including those described in U.S. Pat. Nos. 5,235,033 and 5,034,506. Nucleic acids containing one or more carbocyclic sugars are also included within the definition of nucleic acids (see Jenkins et al. (1995) Chem. Soc. Rev. pp. 169-176), and analogs are also described in, e.g., Rawls, C & E News Jun. 2,1997 page 35. These modifications of the ribose-phosphate backbone may be done to facilitate the addition of additional moieties such as labels, or to alter the stability and half-life of such molecules in physiological environments.

In addition to the naturally occurring heterocyclic bases that are typically found in nucleic acids (e.g., adenine, guanine, thymine, cytosine, and uracil), nucleotide analogs also may include non-naturally occurring heterocyclic bases, such as those described in, e.g., Seela et al. (1999) Helv. Chim. Acta 82:1640. Certain bases used in nucleotide analogs act as melting temperature (Tm) modifiers. For example, some of these include 7-deazapurines (e.g., 7-deazaguanine, 7-deazaadenine, etc.), pyrazolo[3,4-d]pyrimidines, propynyl-dN (e.g., propynyl-dU, propynyl-dC, etc.), and the like. See, e.g., U.S. Pat. No. 5,990,303. Other representative heterocyclic bases include, e.g., hypoxanthine, inosine, xanthine; 8-aza derivatives of 2-aminopurine, 2,6-diaminopurine, 2-amino-6-chloropurine, hypoxanthine, inosine and xanthine; 7-deaza-8-aza derivatives of adenine, guanine, 2-aminopurine, 2,6-diaminopurine, 2-amino-6-chloropurine, hypoxanthine, inosine and xanthine; 6-azacytidine; 5-fluorocytidine; 5-chlorocytidine; 5-iodocytidine; 5-bromocytidine; 5-methylcytidine; 5-propynylcytidine; 5-bromovinyluracil; 5-fluorouracil; 5-chlorouracil; 5-iodouracil; 5-bromouracil; 5-trifluoromethyluracil; 5-methoxymethyluracil; 5-ethynyluracil; 5-propynyluracil, and the like.

A "nucleoside" refers to a nucleic acid component that comprises a base or basic group (comprising at least one homocyclic ring, at least one heterocyclic ring, at least one aryl group, and/or the like) covalently linked to a sugar moiety (a ribose sugar or a deoxyribose sugar), a derivative of a sugar moiety, or a functional equivalent of a sugar moiety (e.g. a carbocyclic ring). For example, when a nucleoside includes a sugar moiety, the base is typically linked to a 1'-position of that sugar moiety. As described above, a base can be a naturally occurring base or a non-naturally occurring base. Exemplary nucleosides include ribonucleosides, deoxyribonucleosides, dideoxyribonucleosides and carbocyclic nucleosides.

A "nucleotide" refers to an ester of a nucleoside, e.g., a phosphate ester of a nucleoside, having one, two, three or more phosphate groups covalently linked to a 5' position of a sugar moiety of the nucleoside.

The terms "polynucleotide" and "oligonucleotide" are used interchangeably. "Oligonucleotide" is a term sometimes used to describe a shorter polynucleotide. An oligonucleotide may be comprised of at least 6 nucleotides, for example at least about 10-12 nucleotides, or at least about 15-30 nucleotides corresponding to a region of the designated nucleotide sequence.

The term "wild-type" as used herein refers to a gene or allele which has the characteristics of that gene or allele when isolated from a naturally occurring source. A wild-type gene or a wild-type allele is that which is most frequently observed in a population and is arbitrarily designated as the "normal" or "wild-type" form of the gene or allele.

In contrast, the term "mutant" or "mutated" refers to a gene or allele which displays modifications in sequence when compared to the wild-type gene or allele. The term "mutation" refers to a change in the sequence of nucleotides of a normally conserved nucleic acid sequence resulting in the formation of a mutant as differentiated from the normal (unaltered) or wild type sequence. Mutations can generally be divided into two general classes, namely, base-pair substitutions (e.g. single nucleotide substitutions) and frame-shift mutations. The latter entail the insertion or deletion of one to several nucleotide pairs.

The term "allele" refers to two sequences which are different by only one or a few bases.

The terms "complementary" or "complementarity" are used in reference to antiparallel strands of polynucleotides related by the Watson-Crick base-pairing rules. The terms "perfectly complementary" or "100% complementary" refer to complementary sequences that have Watson-Crick pairing of all the bases between the antiparallel strands, i.e. there are no mismatches between any two bases in the polynucleotide duplex. However, duplexes are formed between antiparallel strands even in the absence of perfect complementarity. The terms "partially complementary" or "incompletely complementary" refer to any alignment of bases between antiparallel polynucleotide strands that is less than 100% perfect (e.g., there exists at least one mismatch or unmatched base in the polynucleotide duplex). The duplexes between partially complementary strands are generally less stable than the duplexes between perfectly complementary strands.

The term "sample" refers to any composition containing or presumed to contain nucleic acid. This includes a sample of tissue or fluid isolated from an individual for example, skin, plasma, serum, spinal fluid, lymph fluid, synovial fluid, urine, tears, blood cells, organs and tumors, and also to samples of in vitro cultures established from cells taken from an individual, including the formalin-fixed paraffin embedded tissues (FFPET) and nucleic acids isolated therefrom.

The term "primary sequence" refers to the sequence of nucleotides in a polynucleotide or oligonucleotide. Nucleotide modifications such as nitrogenous base modifications, sugar modifications or other backbone modifications are not a part of the primary sequence. Labels, such as chromophores conjugated to the oligonucleotides are also not a part of the primary sequence. Thus two oligonucleotides can share the same primary sequence but differ with respect to the modifications and labels.

The term "primer" refers to an oligonucleotide which hybridizes with a sequence in the target nucleic acid and is capable of acting as a point of initiation of synthesis along a complementary strand of nucleic acid under conditions suitable for such synthesis. As used herein, the term "probe" refers to an oligonucleotide which hybridizes with a sequence in the target nucleic acid and is usually detectably labeled. The probe can have modifications, such as a 3'-terminus modification that makes the probe non-extendable by nucleic acid polymerases, and one or more chromophores. An oligonucleotide with the same sequence may serve as a primer in one assay and a probe in a different assay.

As used herein, the term "bifunctional linker molecule" refers to a compound that can link two or more additional compounds together by chemically interacting with them simultaneously. In the present invention, for example, a bifunctional linker molecule can have one functional domain that is suitable for coupling to a label such as a fluorescent dye and another functional domain or other functional domains capable of coupling to the 5' - terminal position, the 3' -terminal position or an internal position of an oligonucleotide. In the present invention, the bifunctional linker molecule might for example comprise L-threoninol. Various bifunctional linker molecules capable of linking a label into an internal position of an oligonucleotide have been described in U.S. Patent No. 5,585,481, U.S. Patent No. 6,130,323, Nelson et al., Nucl. Acid. Res. 20: 6253-6259, 1992, WO 03/102239 and Ju et al., Proc. Natl. Acad. Sci USA 92: 4347-4351, 1992.

As used herein, the term "target sequence", "target nucleic acid" or "target" refers to a portion of the nucleic acid sequence which is to be either amplified, detected or both.

The terms "hybridized" and "hybridization" refer to the base-pairing interaction of between two nucleic acids which results in formation of a duplex. It is not a requirement that two nucleic acids have 100% complementarity over their full length to achieve hybridization.

The terms "selective hybridization" and "specific hybridization" refer to the hybridization of a nucleic acid predominantly (50% or more of the hybridizing molecule) or nearly exclusively (90% or more of the hybridizing molecule) to a particular nucleic acid present in a complex mixture where other nucleic acids are also present. For example, under typical PCR conditions, primers specifically hybridize to the target nucleic acids to the exclusion of non-target nucleic acids also present in the solution. The specifically hybridized primers drive amplification of the target nucleic acid to produce an amplification product of the target nucleic acid that is at least the most predominant amplification product and is preferably the nearly exclusive (e.g., representing 90% or more of all amplification products in the sample) amplification product. Preferably, the non-specific amplification product is present in such small amounts that it is either non-detectable or is detected in such small amounts as to be easily distinguishable from the specific amplification product. Similarly, probes specifically hybridize to the target nucleic acids to the exclusion of non-target nucleic acids also present in the reaction mixture. The specifically hybridized probes allow specific detection of the target nucleic acid to generate a detectable signal that is at least the most predominant signal and is preferably the nearly exclusive (e.g., representing 90% or more of all amplification products in the sample) signal.

### Allele Specific Probes

Allele-specific hybridization relies on distinguishing between two DNA molecules differing by at least one base by hybridizing an oligonucleotide that is specific for one of the variant sequences to an amplified product obtained from amplifying the nucleic acid sample. An allele-specific assay may also comprise two allele-specific oligonucleotides, e.g., an allele-specific probe for the first variant and an allele-specific probe to the second variant where the probes differentially hybridize to one variant versus the other. Allele-specific hybridization typically employs short oligonucleotides, e.g., 15-35 nucleotides in length. Principles and guidance for designing such probe is available in the art. Hybridization conditions should be sufficiently stringent that there is a significant difference in hybridization intensity between alleles, and preferably an essentially binary response, whereby a probe hybridizes to only one of the alleles. Some probes are designed to hybridize to a segment of target DNA such that the site of interest aligns with a central position of the probe, but this design is not required.

The amount and/or presence of an allele is determined by measuring the amount of allele-specific probe that is hybridized to the sample. Typically, the oligonucleotide is labeled with a label such as a fluorescent label. For example, an allele-specific probe that is specific for an allele of the target nucleic acid is hybridized to nucleic acids obtained from a biological sample under hybridization conditions that result in preferential hybridization to an allele. Fluorescence intensity is measured to determine if specific oligonucleotide has hybridized.

The nucleotide present at the single nucleotide polymorphic site is identified by hybridization under sufficiently stringent hybridization conditions with an oligonucleotide substantially complementary to the allele in a region encompassing the polymorphic site, and exactly complementary to the target allele at this site. Under such sufficiently stringent hybridization conditions, stable duplexes will form only between the probe and the target allele. These probe oligonucleotides can be from about 10 to about 35 nucleotides in length, preferably from about 15 to about 35 nucleotides in length.

The use of substantially, rather than exactly, complementary oligonucleotides may be desirable in assay formats in which optimization of hybridization conditions is limited. For example, in a multi-target immobilized-probe assay format, probes for each target are immobilized on a single solid support. Hybridizations are carried out simultaneously by contacting the solid support with a solution containing target DNA. As all hybridizations are carried out under identical conditions, the hybridization conditions cannot be separately optimized for each probe. The incorporation of mismatches into a probe can be used to adjust duplex stability when the assay format precludes adjusting the hybridization conditions. The effect of a particular introduced mismatch on duplex stability is well known, and the duplex stability can be routinely both estimated and empirically determined, as described above. Suitable hybridization conditions, which depend on the exact size and sequence of the probe, can be selected empirically using the guidance provided herein and well known in the art. The use of oligonucleotide probes to detect single base pair differences in sequence is described in, for example, Conner et al., 1983, Proc. Natl. Acad. Sci. USA 80:278-282, and U.S. Pat. Nos. 5,468,613 and 5,604,099.

The proportional change in stability between a perfectly matched and a single-base mismatched hybridization duplex depends on the length of the hybridized oligonucleotides. Duplexes formed with shorter probe sequences are destabilized proportionally more by the presence of a mismatch. In practice, oligonucleotides between about 15 and about 35 nucleotides in length are preferred for sequence-specific detection. Furthermore, because the ends of a hybridized oligonucleotide undergo continuous random dissociation and re-annealing due to thermal energy, a mismatch at either end destabilizes the hybridization duplex less than a mismatch occurring internally Preferably, for discrimination of a single base pair change in target sequence, the probe sequence is selected which hybridizes to the target sequence such that the mutation site occurs in the interior region of the probe.

### 5'-Nuclease Assay

The detection of a target nucleic acid can be performed using a "TaqMan^{®}" or "5'-nuclease assay", as described in U.S. Pat. Nos. 5,210,015; 5,487,972; and 5,804,375; and Holland et al., 1988, Proc. Natl. Acad. Sci. USA 88:7276-7280. In the TaqMan^{®} assay, labeled detection probes that hybridize within the amplified region are present during the amplification reaction. The probes are modified so as to prevent the probes from acting as primers for DNA synthesis. The amplification is performed using a DNA polymerase having 5' to 3' exonuclease activity. During each synthesis step of the amplification, any probe which hybridizes to the target nucleic acid downstream from the primer being extended is degraded by the 5' to 3' exonuclease activity of the DNA polymerase. Thus, the synthesis of a new target strand also results in the degradation of a probe, and the accumulation of degradation product provides a measure of the synthesis of target sequences.

Any method suitable for detecting degradation product can be used in a 5' nuclease assay. Often, the detection probe is labeled with two fluorescent dyes, one of which is capable of quenching the fluorescence of the other dye. The dyes are attached to the probe, typically with the reporter or detector dye attached to the 5' terminus and the quenching dye attached to an internal site, such that quenching occurs when the probe is in an unhybridized state and such that cleavage of the probe by the 5' to 3' exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Pat. Nos. 5,491,063 and 5,571,673 describe alternative methods for detecting the degradation of probe which occurs concomitant with amplification.

A 5' nuclease assay for the detection of a target nucleic acid can employ any polymerase that has a 5' to 3' exonuclease activity. Thus, in some embodiments, the polymerases with 5'-nuclease activity are thermostable and thermoactive nucleic acid polymerases. Such thermostable polymerases include, but are not limited to, native and recombinant forms of polymerases from a variety of species of the eubacterial genera *Thermus*, *Thermatoga*, and *Thermosipho*, as well as chimeric forms thereof For example, *Thermus* species polymerases that can be used in the methods of the invention include *Thermus aquaticus (Taq)* DNA polymerase, *Thermus thermophilus (Tth)* DNA polymerase, *Thermus* species Z05 (Z05) DNA polymerase, *Thermus* species sps17 (sps17), and *Thermus* species Z05 (e.g., described in U.S. Pat. Nos. 5,405,774; 5,352,600; 5,079,352; 4,889,818; 5,466,591; 5,618,711; 5,674,738, and 5,795,762). *Thermatoga* polymerases that can be used in the methods of the invention include, for example, *Thermatoga maritima* DNA polymerase and *Thermatoga neapolitana* DNA polymerase, while an example of a *Thermosipho* polymerase that can be used is *Thermosipho africanus* DNA polymerase. The sequences of *Thermatoga maritima* and *Thermosipho africanus* DNA polymerases are published in International Application WO 92/06200. The sequence of *Thermatoga neapolitana* may be found in International Application WO 97/09451.

In the 5' nuclease assay, the amplification detection is typically concurrent with amplification (i.e., "real-time"). In some embodiments the amplification detection is quantitative, and the amplification detection is real-time. In some embodiments, the amplification detection is qualitative (e.g., end-point detection of the presence or absence of a target nucleic acid). In some embodiments, the amplification detection is subsequent to amplification. In some embodiments, the amplification detection is qualitative, and the amplification detection is subsequent to amplification.

The probe can be labeled with any number of labels, but is typically a fluorescent label. In some embodiments, the fluorophore moiety is selected from the group consisting of fluorescein-family dyes, polyhalofluorescein-family dyes, hexachlorofluorescein-family dyes, coumarin-family dyes, rhodamine-family dyes, cyanine-family dyes, oxazine-family dyes, thiazin-family dyes, squaraine-family dyes, chelated lanthanide-family dyes, azo-family dyes, triphenylmethane-family dyes, and BODIPY^{®}-family dyes.

The assay often comprises a probe labeled with a fluorescent label and a quencher moiety. In some embodiments, the quencher moiety is selected from the group consisting of fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, BODIPY^{®}-family dyes, azo-family dyes; triphenylmethane-family dyes, low-fluorescent quencher moieties (i.e., "dim donors") and non-fluorescent quencher moieties (e.g., so-called "dark quenchers" including Black Hole Quenchers™ (BHQ)).

### Rhodamine-derived dyes

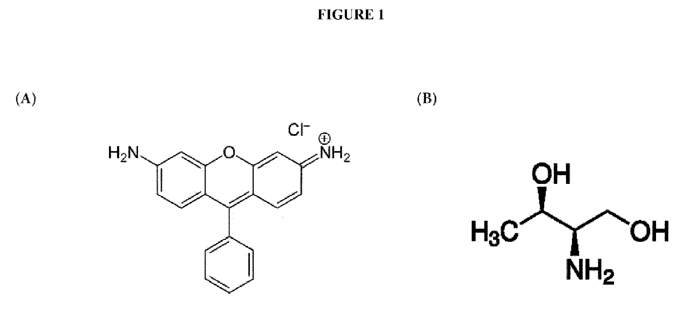

Rhodamine is a fluorescent dye that contains a core structure as shown in Figure 1A. Several derivatives of rhodamine, such as Carboxytetramethylrhodamine (TAMRA) and sulforhodamine 101 acid chloride (Texas Red) are also fluorescent dyes, commonly used for imaging purposes. Novel rhodamine derivatives whose fluorescence emission maxima are in the range of 600 nm have been disclosed in U.S. Patent No. 6,184,379, ('379). These derivatives have structures that can be represented by the following formula: 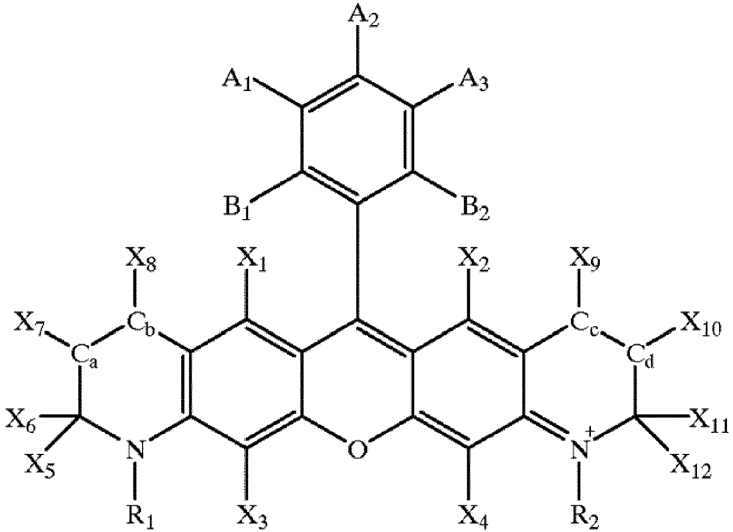 in which Ca, Cb, Cc and Cd each denote a C atom, Ca and Cb are either linked together by a single bond or by a double bond, and Cc and Cd are either linked together by a single bond or by a double bond; X1, X2, X3, X4, X7 and X10 are hydrogen and X5, X6, X8, X9, X11 and X12 are methyl;

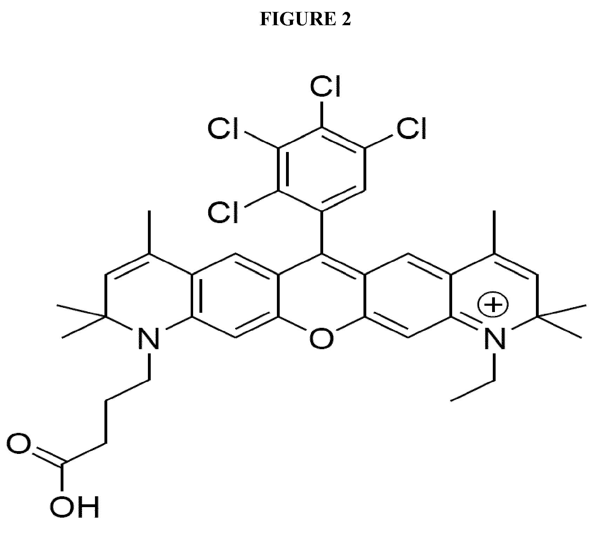

R1 and R2 are either identical or different and are selected from a group consisting of hydrogen and alkyl with 1-20 C atoms, wherein the alkyl residues are optionally substituted by at least one hydroxyl, halogen, sulfonic acid, amino, carboxy or alkoxycarbonyl group, and at least R1 contains an activatable group; A1, A2, A3, B1 are either chlorine or fluorine and B2 is either chlorine, fluorine or hydrogen. Two particular compounds, JA270 and JF9 are also described in the '379 patent, with a carboxylic acid analog of JA270 (Figure 2) shown to be particularly suitable as a fluorophore that can be used as in a fluorescence resonance energy transfer (FRET) system.

A particularly preferred use of JA270 would be as a fluorophore attached to an oligonucleotide probe in a TaqMan^{®} assay that can be quenched by an appropriate quencher molecule that is also attached to the probe. Furthermore, in order to maximize the utility of JA270 as a fluorescent label, it would be desirable to attach a bifunctional linker to JA270 that would allow placement of the label anywhere within the probe oligonucleotide sequence. A suitable bifunctional linker would be L-threoninol or (2*R*,3*R*)-2-amino-1,3-butanediol (Figure 1B), that can form an amide bond with JA270, and can be attached at both hydroxyl moieties with standard reactive groups for oligonucleotide synthesis such as phosphoramidite and blocking groups such as 4,4'dimethyoxytrityl (DMT). As described in the present invention, the placement of the JA270 dye in the internal site of the probe oligonucleotide would exhibit unexpected improvement in the stability and function of the probe.

### Polymorphisms in the Apolipoprotein E (apoE) gene

ApoE is a major component of various lipoprotein species and plays a central role in the control of plasma cholesterol levels. The gene encoding apoE is present in heterogeneous forms, some of which are known to differentially affect transcriptional activity or to encode structurally and functionally distinct protein isoforms. The three major isoforms of apoE, named apoE2, apoE3, and apoE4, have different binding affinities for the low-density lipoprotein (LDL) receptor, and are associated with different levels of plasma lipid and lipoprotein. The three major isoforms are characterized by the presence of cystine (Cys) for the apoE2 and apoE3 forms and arginine (Arg) for the apoE4 form at position 112 of the apoE polypeptide chain and Cys for the apoE2 form and Arg for the apoE3 and apoE4 forms at position 158. The variability of amino acids 112 and 158 is based on single nucleotide polymorphisms (SNPs) present at nucleotide positions 334 and 472, respectively, of the apoE gene. At either location, Cys is determined by the codon TGC and Arg is determined by the codon CGC. These alleles are referred herein as the 334T/C and the 472 T/C alleles. The combinations, 334T/472T, 334T/472C and 334C/472C form the known isoform-specific apoE alleles, ε2, ε3, and ε4, respectively.

Interest in apoE genotyping has been high due to its recognition as providing valuable information in identifying individuals at risk for cardiovascular and neurological diseases. In particular, the presence of the apoE allele ε4 was found to be associated with the pathogenesis of peripheral and coronary artery disease, as well as neurodegenerative disorders including the sporadic and late-onset familial forms of Alzheimers's disease. Methods commonly used for genotyping include PCR-Restriction Fragment Length Polymorphism (PCR-RFLP) analysis and PCR followed by sequencing or mass spectrometry, but both are time-consuming and low-throughput methods. Genotyping by real-time PCR using either allele-specific primers or allele-specific probes have also been described (Calero et al., J. Neurosci Methods. 2009, 183(2):238-40; Koch et al., Clin. Chem. Lab Med. 2002, 40:1123-1131) and have shown to be quick and effective alternatives. A need therefore exists to develop reagents used in real-time PCR assays, such as the TaqMan^{®} technology, that can both quickly and accurately genotype the apoE alleles.

The following examples and figures are provided to aid the understanding of the present invention, the true scope of which is set forth in the appended claims. It is understood that modifications can be made in the procedures set forth without departing from the invention.

### EXAMPLES

### Example 1 Analysis of a JA270-labeled control oligonucleotide used for calibration

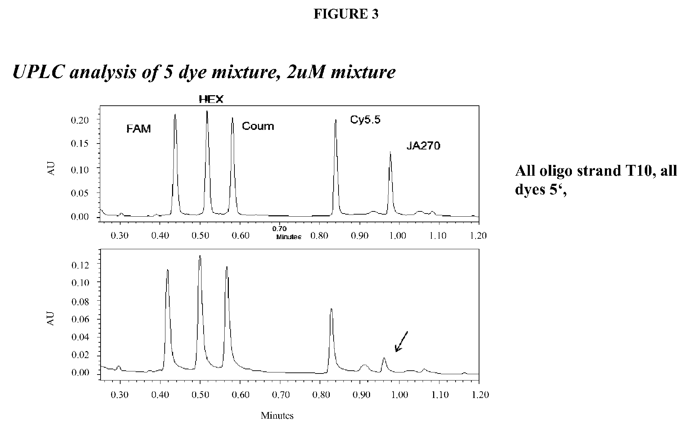

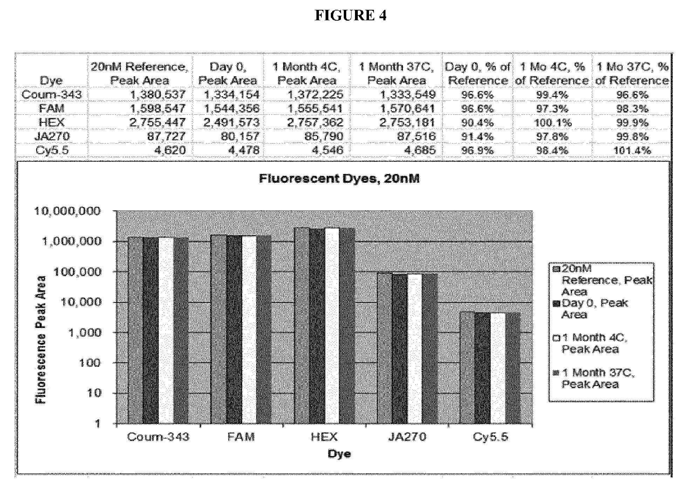

A reference reagent solution to be used for calibration of fluorescence detection was produced and contained five 10-mer deoxythymidine (dT) oligonucleotides at 2µM concentration all labeled at the 5' terminal nucleotide using five different fluorescent dyes. The five fluorescent dyes were FAM, HEX, JA270, Coumarin-343, and Cy5.5. The purity and stability of this reference solution was analyzed by a Waters Acquity UPLC System with Photodiode Array (PDA) and fluorescence detection. Chromatography was performed on an Acquity OST C8 1.7µm particle column. The mobile phases consist of 0.1M Hexylammonium acetate (HAA) pH 7.0 as Buffer A and 100% acetonitrile as Buffer B and the oligonucleotides were separated across a gradient of 30-60% Buffer B for the first 0.6 minutes, and followed by 60-95% Buffer B for 1.4 minutes, at a flow rate of 1mL/min. The elution profiles of the oligonucleotides for an experiment performed in the morning (top) and evening (bottom) of the same day is shown on Figure 3. Interestingly, the JA270 peak was much reduced in size in the evening run, suggesting possible issues with stability of this particular oligonucleotide. It was suspected that due to the size and hydrophobicity of the JA270 dye, this particular oligonucleotide would fall out of solution. In order to decrease hydrophobicity of JA270-labeled oligonucleotide, it was determined to synthesize the JA270-labeled 10-mer dT with the JA270 placed in an internal position instead of at the 5' terminus. The internal placement of the JA270 dye was made possible by attaching the L-threoninol linker to the carboxylic acid moiety of the dye and adding a reactive phosphoramidite group that enables linkage with an internal phosphate within the oligonucleotide. UPLC analysis was performed again and the results are shown on the table and bar graph depicted in Figure 4. By placing the JA270 dye in the internal site of the oligonucleotide, stability of the JA270-labeled 10-mer dT could be maintained even after one month of storage.

### Example 2 Analysis of a JA270-labeled probe for apoE genotyping

An assay for genotyping the ε2, ε3, and ε4 alleles of the apoE gene was developed using TaqMan^{®} technology and allele-specific TaqMan^{®} probe oligonucleotides. A "mastermix" oligonucleotide solution was prepared and contained the following probes:
5'-end FAM labeled 22-mer probe selective for the 334T allele
5'-end HEX labeled 22-mer probe selective for the 334C allele
5'-end JA270 labeled 22-mer probe selective for the 472C allele
5'-end Cy5.5 labeled 22-mer probe selective for the 472T allele

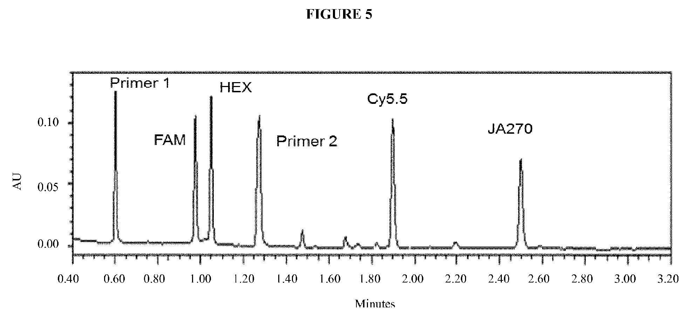

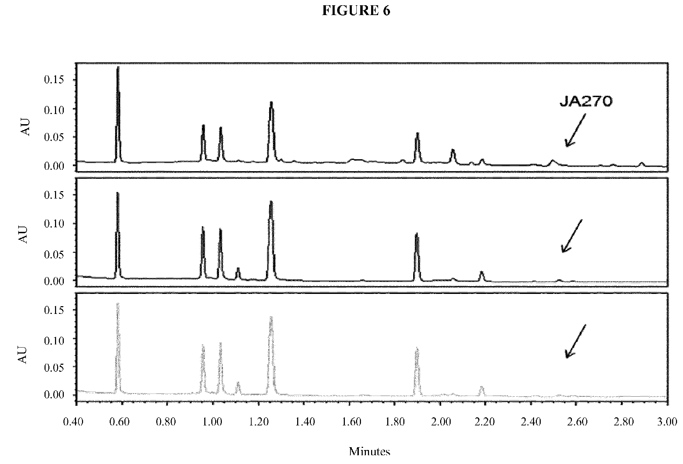

The "mastermix" solution was analyzed for correct composition and stability by UPLC using Acquity OST C18 1.7µm particle column . The mobile phases consist of 0.1M Triethylammonium acetate (TEAA) pH 7.0 as Buffer A and 100% acetonitrile as Buffer B and the oligonucleotides were separated across a gradient of 5-60% Buffer B for 3 minutes at a flow rate of 1 mL/min. The elution profile of a "fresh" solution at Day 0 is shown on Figure 5 where the oligonucleotide peaks for all four probes and the two PCR primers could be observed. However, as seen in Figure 6, when this solution was analyzed after three days of storage at 4°C, the peak for the JA270-labeled oligonucleotide had disappeared. Similar to what was believed to be the reason for the instability of the JA270-labeled reference dT oligonucleotide labeled at the 5' terminus in Example 1, the instability of the JA-270 labeled apoE probe was also believed to be due to aggregation of the oligonucleotide and its falling out of solution.

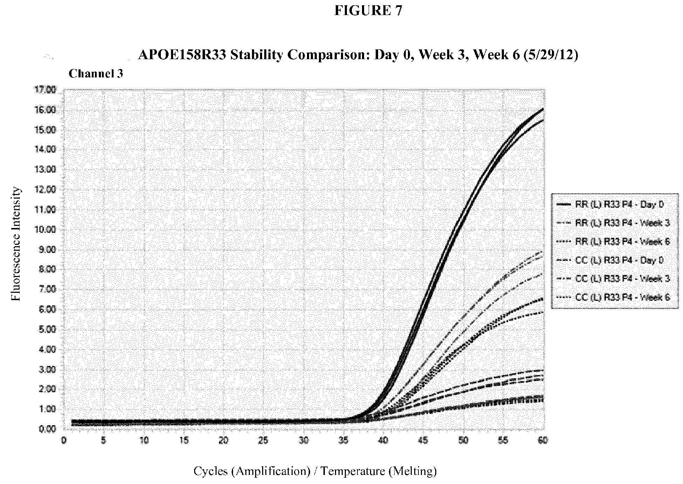

To solve the instability problem, the length of the JA270-labeled probe was first increased from a 22-mer oligonucleotide to a 25-mer oligonucleotide. One version of the 25-mer probe still placed JA270 at the 5' terminus (designated probe R33) whereas in another version, the JA270 dye was now placed internally between nucleotides 12 and 13 (designated probe R34). Internal placement of the dye was made possible by attaching the L-threoninol linker to the carboxylic acid moiety on JA270. A real-time PCR assay was then performed using both probes R33 and R44 under buffers and conditions that are listed in Tables 1 and 2 below. A positive control plasmid template carrying a 472C allele and a negative control plasmid template carrying a 472T allele were used to compare the performance of the R33 and R34 probes to generate allele-specific PCR growth curves. The R34 probe with the JA270 dye placed internally showed good stability with no significant change in growth curves at using the probe stored for either three weeks or six weeks. In contrast, as shown in Figure 7, the R33 probe labeled at the 5' terminus displayed a huge decline in the fluorescence signals in growth curve generated by the probe that had been stored for three weeks or six weeks. This result clearly showed that placing the JA270 dye in an internal site within the probe oligonucleotide greatly increased the stability and the performance of the probe.

**TABLE 1**

| **R2: Master Mix** | **Prototype II** | | | |
|---|---|---|---|---|
| **Component** | **Conc. inMMx (2.79x)** | | **Conc. inPCR (final)** | |
| Tricine pH 8.3 (mM) | 1674 | mM | 60 | mM |
| KOH (mM) | 38.8 | mM | 13.9 | mM |
| EDTA (mM) | 0.56 | mM | 02 | mM |
| Glycerol (%) | 41.9 | % | 15 | % |
| Tween 20 (%) | 0.056 | % | 0.02 | % |
| DMSO (%) | 5.6 | % | 2 | % |
| Na Azide (%) | 0.09* | % | N/A | % |
| dATP (mM) | 0.56 | mM | 0.2 | mM |
| dCTP (mM) | 0.56 | mM | 0.2 | mM |
| dGTP (mM) | 0.56 | mM | 0.2 | mM |
| dUTP (mM) | 1.12 | mM | 0.4 | mM |
| dTTP (mM) | 0.14 | mM | 0.05 | mM |
| Z05 (Unit/uL) | 0.84 | U/uL | 15 | U (or 0.30 U/uL) |
| Aptamer, 46A (uM) | 0.56 | uM | 0.2 | uM |
| AmpErase UNG (Unit/uL) | 0.167 | U/uL | 3 | U (or 0.060 U/uL) |
| 5'APOE5P05 (uM) | 1.12 | uM | 0.4 | uM |
| 3'APOE3P04 (uM) | 1.12 | uM | 0.4 | uM |
| Probe APOE112C07 (uM) FAM | 0.84 | uM | 0.3 | uM |
| Probe APOE112R05 (uM) HEX | 0.84 | uM | 0.3 | uM |
| Probe APOE158R28 (uM) JA270 | 0.84 | uM | 0.3 | uM |
| Probe APOE158C14 (uM) CY5.5 | 0.56 | uM | 0.2 | uM |
| | * Concentration in Master Mix | | | |

| **R1: Cation/Cofactor/KOAc** | **Prototype II** | | | |
|---|---|---|---|---|
| **Component** | **Conc. in MMx (7.04x)** | | **Conc. in PCR (final)** | |
| Mg (OAc)2, pH 6.5 | 14.1 | mM | 2 | mM |
| Mn (OAc)2, pH 6.3 | 7.04 | mM | 1 | mM |
| KOAc, pH 8.0 (mM) | 634 | mM | 90 | mM |

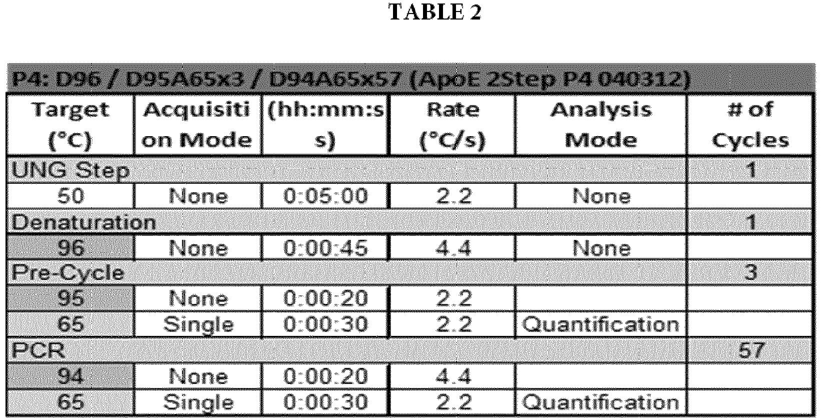

## Claims

1. A method of making a labeled oligonucleotide for use as a hybridization probe in a PCR reaction comprising:
(a) providing a fluorescent dye that is a rhodamine derivative wherein said fluorescent dye is attached with a bifunctional linker molecule;
(b) binding a reactive group to the fluorescent dye via the bifunctional linker molecule for incorporating said fluorescent dye within the oligonucleotide;
(c) incorporating the fluorescent dye between two internal nucleotides of the oligonucleotide, with the proviso that the fluorescent dye is not incorporated at the 5' terminus of the oligonucleotide,
wherein the bifunctional linker molecule is consisting of L-threoninol or (2R,3R)-2-amino-1,3-butanediol.

2. The method of claim 1 wherein the fluorescent dye is a compound with the general formula 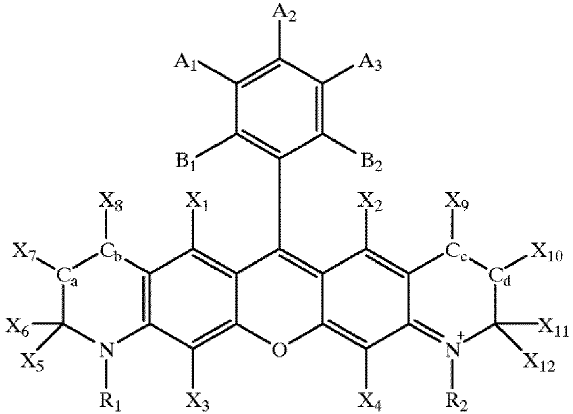
in which Ca, Cb, Cc and Cd each denote a C atom, Ca and Cb are either linked together by a single bond or by a double bond, and Cc and Cd are either linked together by a single bond or by a double bond;
X1, X2, X3, X4, X7 and X10 are hydrogen and X5, X6, X8, X9, X11 and X12 are methyl;
R1and R2 are either identical or different and are selected from a group consisting of hydrogen and alkyl with 1-20 C atoms, wherein the alkyl residues are optionally substituted by at least one hydroxyl, halogen, sulfonic acid, amino, carboxy or alkoxycarbonyl group, and at least R1 contains an activatable group;
A1, A2, A3, B1 are either chlorine or fluorine and B2 is either chlorine, fluorine or hydrogen.

3. The method of claim 2 wherein the fluorescent dye is:

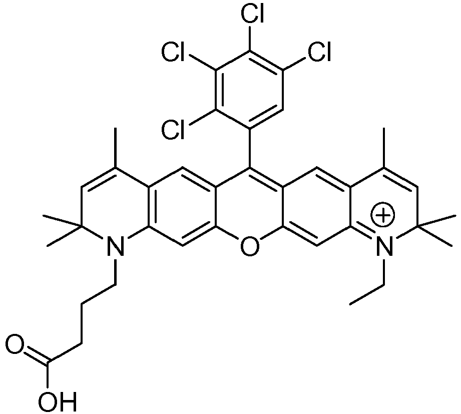

4. The method of claim 1 wherein the labeled oligonucleotide further comprises a quencher molecule attached at the 5' terminus of the labeled oligonucleotide.

5. A method for detecting the presence or absence of a target nucleic acid or a target allele of a nucleic acid in a test sample, comprising:
performing a PCR reaction with the use of a labeled probe oligonucleotide that hybridizes with the target nucleic acid, wherein the labeled probe oligonucleotide is **characterized by** having:
a fluorescent dye that is a rhodamine derivative which is attached with a bifunctional linker molecule; and
a reactive group bound to the bifunctional linker molecule for incorporating the fluorescent dye between two internal nucleotides of the oligonucleotide, with the proviso that that the fluorescent dye is not incorporated at the 5' terminus of the oligonucleotide, wherein the bifunctional linker molecule is consisting of L-threoninol or (2R,3R)-2-amino-1,3-butanediol;
detecting the signal from the fluorescent dye wherein the intensity of said signal represents the presence or absence of the target nucleic acid.

6. The method of claim 5 wherein the fluorescent dye is a compound with the general formula 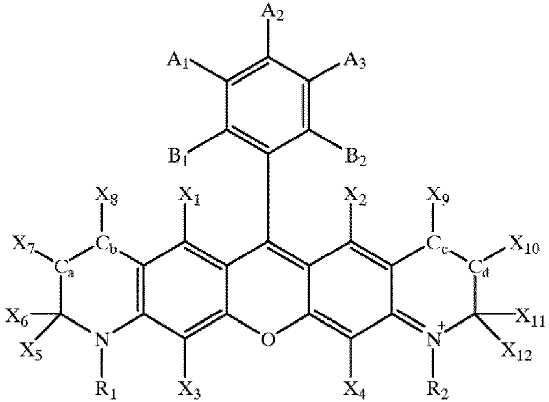
In which Ca, Cb, Cc and Cd each denote a C atom, Ca and Cb are either linked together by a single bond or by a double bond, and Cc and Cd are either linked together by a single bond or by a double bond;
X1, X2, X3, X4, X7 and X10 are hydrogen and X5, X6, X8, X9, X11 and X12 are methyl; \
R1and R2 are either identical or different and are selected from a group consisting of hydrogen and alkyl with 1-20 C atoms, wherein the alkyl residues are optionally substituted by at least one hydroxyl, halogen, sulfonic acid, amino, carboxy or alkoxycarbonyl group, and at least R1 contains an activatable group;
A1, A2, A3, B1 are either chlorine or fluorine and B2 is either chlorine, fluorine or hydrogen.

7. The method of claim 6 wherein the fluorescent dye is:

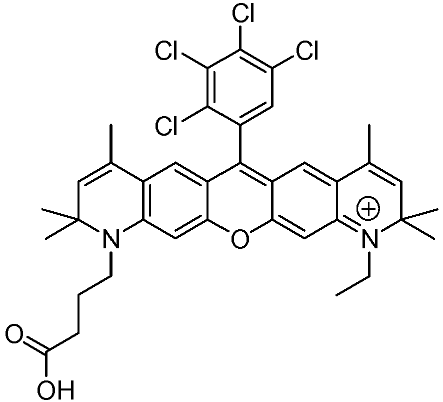

8. The method of claim 5 wherein the labeled oligonucleotide further comprises a quencher molecule attached at the 5' terminus of the labeled oligonucleotide.

9. The method of claim 5 wherein the target nucleic acid is the apolipoprotein E gene.

10. The method of claim 5 wherein the target allele of a nucleic acid is the 334T/C allele or the 472 T/C allele of the apolipoprotein E gene.

## Patentansprüche

1. Verfahren zur Herstellung eines markierten Oligonukleotids zur Verwendung als Hybridisierungssonde in einer PCR-Reaktion, das Folgendes umfasst:
(a) Bereitstellen eines Fluoreszenzfarbstoffs, bei dem es sich um ein Rhodaminderivat handelt, wobei der Fluoreszenzfarbstoff mit einem bifunktionellen Linkermolekül verbunden ist;
(b) Binden einer reaktionsfähigen Gruppe an den Fluoreszenzfarbstoff über das bifunktionelle Linkermolekül zum Einbauen des Fluoreszenzfarbstoffs in das Oligonukleotid;
(c) Einbauen des Fluoreszenzfarbstoffs zwischen zwei interne Nukleotide des Oligonukleotids, mit der Maßgabe, dass der Fluoreszenzfarbstoff nicht am 5'-terminalen Ende des Oligonukleotids eingebaut wird,
wobei das bifunktionelle Linkermolekül aus L-Threoninol oder (2R,3R)-2-Amino-1,3-butandiol besteht.

2. Verfahren nach Anspruch 1, wobei es sich bei dem Fluoreszenzfarbstoff um eine Verbindung der allgemeinen Formel 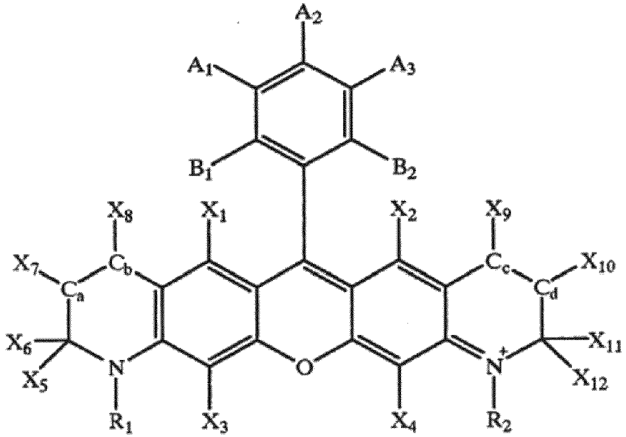
handelt, in der Ca, Cb, Cc und Cd jeweils ein C-Atom bedeuten, Ca und Cb miteinander entweder über eine einfache Bindung oder über eine Doppelbindung verknüpft sind und Cc und Cd miteinander entweder über eine einfache Bindung oder über eine Doppelbindung verknüpft sind;
X1, X2, X3, X4, X7 und X10 Wasserstoff bedeuten und X5, X6, X8, X9, X11 und X12 Methyl bedeuten;
R1 und R2 entweder gleich oder verschieden sind und aus einer Gruppe bestehend aus Wasserstoff und Alkyl mit 1-20 C-Atomen ausgewählt sind, wobei die Alkylreste gegebenenfalls durch mindestens eine Hydroxyl-, Halogen-, Sulfonsäure-, Amino-, Carboxyl- oder Alkoxycarbonylgruppe substituiert sind und zumindest R1 eine aktivierbare Gruppe enthält;
A1, A2, A3, B1 entweder Chlor oder Fluor bedeuten und B2 entweder Chlor, Fluor oder Wasserstoff bedeutet.

3. Verfahren nach Anspruch 2, wobei es sich bei dem Fluoreszenzfarbstoff um: 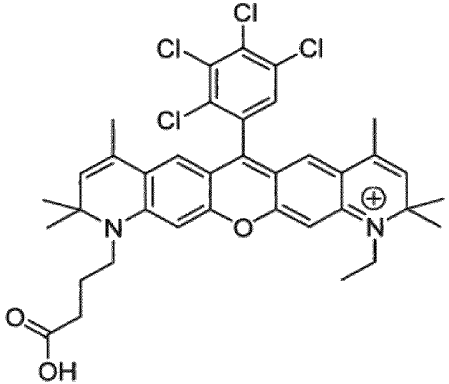 handelt.

4. Verfahren nach Anspruch 1, wobei das markierte Oligonukleotid weiterhin ein am 5'-terminalen Ende des markierten Oligonukleotids gebundenes Quencher-Molekül umfasst.

5. Verfahren zum Nachweisen des Vorliegens bzw. Fehlens einer Zielnukleinsäure oder eines Zielallels einer Nukleinsäure in einer Testprobe, das Folgendes umfasst:
Durchführen einer PCR-Reaktion unter Verwendung eines markierten Sondenoligonukleotids, das mit der Zielnukleinsäure hybridisiert, wobei das markierte Sondenoligonukleotid **dadurch gekennzeichnet ist, dass** es Folgendes aufweist:
einen Fluoreszenzfarbstoff, bei dem es sich um ein Rhodaminderivat handelt, wobei der Fluoreszenzfarbstoff mit einem bifunktionellen Linkermolekül verbunden ist; und
eine reaktionsfähige Gruppe, die an das bifunktionelle Linkermolekül gebunden ist, um den Fluoreszenzfarbstoff zwischen zwei interne Nukleotide des Oligonukleotids einzubauen, mit der Maßgabe, dass der Fluoreszenzfarbstoff nicht am 5'-terminalen Ende des Oligonukleotids eingebaut wird, wobei das bifunktionelle Linkermolekül aus L-Threoninol oder (2R,3R)-2-Amino-1,3-butandiol besteht;
Nachweisen des Signals von dem Fluoreszenzfarbstoff, wobei die Intensität des Signals das Vorhandensein bzw. Fehlen der Zielnukleinsäure darstellt.

6. Verfahren nach Anspruch 5, wobei es sich bei dem Fluoreszenzfarbstoff um eine Verbindung der allgemeinen Formel 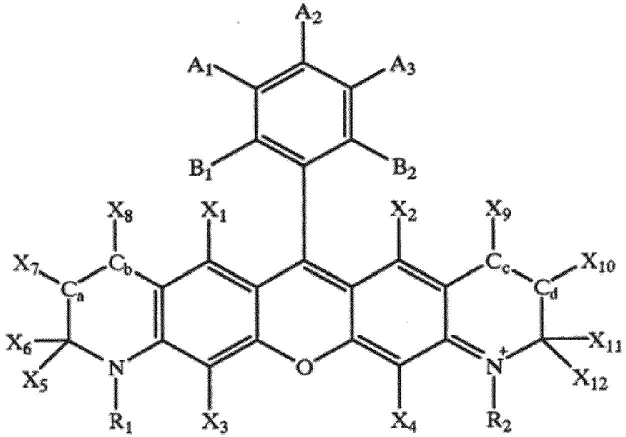
handelt, in der Ca, Cb, Cc und Cd jeweils ein C-Atom bedeuten, Ca und Cb miteinander entweder über eine einfache Bindung oder über eine Doppelbindung verknüpft sind und Cc und Cd miteinander entweder über eine einfache Bindung oder über eine Doppelbindung verknüpft sind;
X1, X2, X3, X4, X7 und X10 Wasserstoff bedeuten und X5, X6, X8, X9, X11 und X12 Methyl bedeuten;
R1 und R2 entweder gleich oder verschieden sind und aus einer Gruppe bestehend aus Wasserstoff und Alkyl mit 1-20 C-Atomen ausgewählt sind, wobei die Alkylreste gegebenenfalls durch mindestens eine Hydroxyl-, Halogen-, Sulfonsäure-, Amino-, Carboxyl- oder Alkoxycarbonylgruppe substituiert sind und zumindest R1 eine aktivierbare Gruppe enthält;
A1, A2, A3, B1 entweder Chlor oder Fluor bedeuten und B2 entweder Chlor, Fluor oder Wasserstoff bedeutet.

7. Verfahren nach Anspruch 6, wobei es sich bei dem Fluoreszenzfarbstoff um 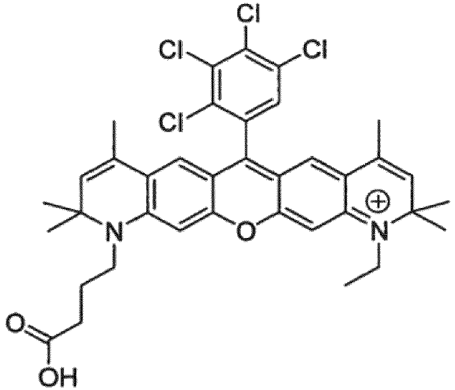 handelt.

8. Verfahren nach Anspruch 5, wobei das markierte Oligonukleotid weiterhin ein am 5'-terminalen Ende des markierten Oligonukleotids gebundenes Quencher-Molekül umfasst.

9. Verfahren nach Anspruch 5, wobei es sich bei der Zielnukleinsäure um das Apolipoprotein-E-Gen handelt.

10. Verfahren nach Anspruch 5, wobei es sich bei dem Zielallel einer Nukleinsäure um das 334T/C-Allel oder das 472T/C-Allel des Apolipoprotein-E-Gens handelt.

## Revendications

1. Procédé de préparation d'un oligonucléotide marqué pour une utilisation comme sonde d'hybridation dans une réaction PCR comprenant :
(a) prendre un colorant fluorescent qui est un dérivé de rhodamine, ledit colorant fluorescent étant fixé à une molécule de liaison difonctionnelle ;
(b) lier un groupe réactif au colorant fluorescent via la molécule de liaison difonctionnelle pour incorporer ledit colorant fluorescent dans l'oligonucléotide ;
(c) incorporer le colorant fluorescent entre deux nucléotides internes de l'oligonucléotide, à condition que le colorant fluorescent ne soit pas incorporé au niveau de l'extrémité 5' de l'oligonucléotide,
la molécule de liaison difonctionnelle étant constituée de L-thréoninol ou de (2R,3R)-2-amino-1,3-butanediol.

2. Procédé selon la revendication 1, le colorant fluorescent étant un composé de formule générale 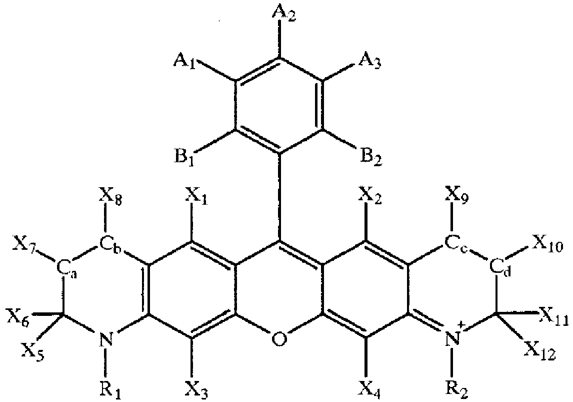
dans laquelle Cₐ, C_{b}, C_{c} et C_{d} représentent, chacun, un atome de C, Cₐ et C_{b} sont liés ensemble soit par une simple liaison, soit par une double liaison et C_{c} et C_{d} sont liés ensemble soit par une simple liaison, soit par une double liaison ;
X₁, X₂, X₃, X₄, X₇ et X₁₀ représentent hydrogène et X₅, X₆, X₈, X₉, X₁₁ et X₁₂ représente méthyle ;
R₁ et R₂ sont soit identiques, soit différents et sont choisis dans un groupe constitué par l'hydrogène et alkyle comprenant 1-20 atomes de C, les résidus alkyle étant éventuellement substitués par au moins un groupe hydroxyle, halogène, acide sulfonique, amino, carboxy ou alcoxycarbonyle, et au moins R₁ contenant un groupe activable ;
A₁, A₂, A₃, B₁ représentent soit chlore, soit fluor et B₂ représente soit chlore, soit fluor, soit hydrogène.

3. Procédé selon la revendication 2, le colorant fluorescent étant

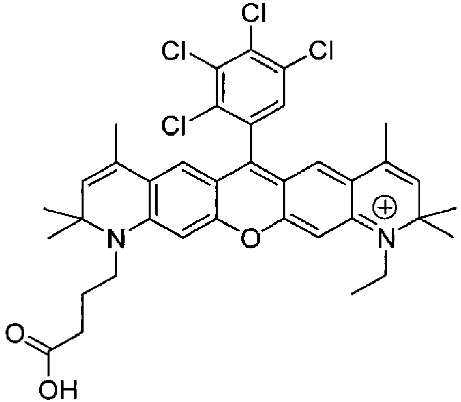

4. Procédé selon la revendication 1, l'oligonucléotide marqué comprenant en outre une molécule d'extinction fixée en l'extrémité 5' de l'oligonucléotide marqué.

5. Procédé pour détecter la présence ou l'absence d'un acide nucléique cible ou d'un allèle cible d'un acide nucléique dans un échantillon d'analyse, comprenant :
réaliser une réaction PCR à l'aide d'un oligonucléotide sonde marqué qui s'hybride avec l'acide nucléique cible, l'oligonucléotide sonde marqué étant **caractérisé en ce qu'**il présente :
un colorant fluorescent qui est un dérivé de rhodamine qui est fixé à une molécule de liaison difonctionnelle ; et
un groupe réactif lié à la molécule de liaison difonctionnelle pour incorporer le colorant fluorescent entre deux nucléotides internes de l'oligonucléotide, à condition que le colorant fluorescent ne soit pas incorporé au niveau de l'extrémité 5' de l'oligonucléotide, la molécule de liaison difonctionnelle étant constituée de L-thréoninol ou de (2R,3R)-2-amino-1,3-butanediol ;
détecter le signal du colorant fluorescent, l'intensité dudit signal représentant la présence ou l'absence de l'acide nucléique cible.

6. Procédé selon la revendication 5, le colorant fluorescent étant un composé de formule générale 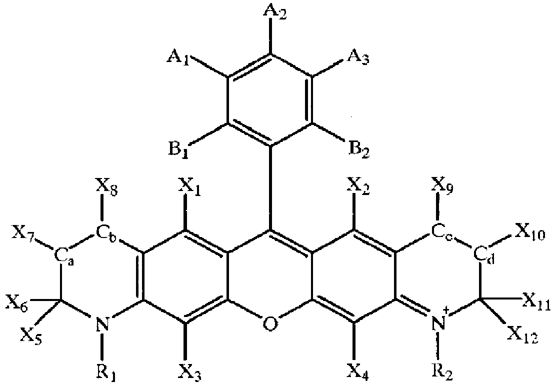
dans laquelle Cₐ, C_{b}, C_{c} et C_{d} représentent, chacun, un atome de C, Cₐ et C_{b} sont liés ensemble soit par une simple liaison, soit par une double liaison et C_{c} et C_{d} sont liés ensemble soit par une simple liaison, soit par une double liaison ;
X₁, X2, X₃, X₄, X₇ et X₁₀ représentent hydrogène et X₅, X₆, X₈, X₉, X₁₁ et X₁₂ représentent méthyle ;
R₁ et R₂ sont soit identiques, soit différents et sont choisis dans un groupe constitué par l'hydrogène et alkyle comprenant 1-20 atomes de C, les résidus alkyle étant éventuellement substitués par au moins un groupe hydroxyle, halogène, acide sulfonique, amino, carboxy ou alcoxycarbonyle, et au moins R₁ contenant un groupe activable ;
A₁, A₂, A₃, B₁ représentent soit chlore, soit fluor et B₂ représente soit chlore, soit fluor, soit hydrogène.

7. Procédé selon la revendication 6, le colorant fluorescent étant

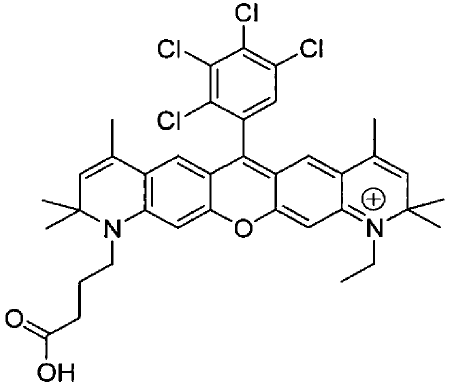

8. Procédé selon la revendication 5, l'oligonucléotide marqué comprenant en outre une molécule d'extinction fixée en l'extrémité 5' de l'oligonucléotide marqué.

9. Procédé selon la revendication 5, l'acide nucléique cible étant le gène de l'apolipoprotéine E.

10. Procédé selon la revendication 5, l'allèle cible d'un acide nucléique étant l'allèle 334T/C ou l'allèle 472 T/C du gène de l'apolipoprotéine E.
